(19) Europäisches Patentamt
European Patent Office
Office européen des brevets

(11) **EP 3 369 402 A1**

(12) **EUROPEAN PATENT APPLICATION**

(43) Date of publication:
**05.09.2018 Bulletin 2018/36**

(51) Int Cl.:
*A61F 2/966* *(2013.01)*        *A61F 2/95* *(2013.01)*

(21) Application number: **18275026.5**

(22) Date of filing: **19.02.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **28.02.2017   US 201762464476 P**

(71) Applicant: **Cook Medical Technologies LLC Bloomington, IN 47404 (US)**

(72) Inventors:
• **WILGER, Kevin D.**
  **Lafayette, IN Indiana 47909 (US)**
• **Bradway, Ryan C.**
  **West Lafayette, IN Indiana 47906 (US)**
• **KRATZBERG, Jarin**
  **Lafayette, IN Indiana 47909 (US)**
• **Davis, Brandon J.**
  **Fishers, IN Indiana 46037 (US)**

(74) Representative: **Williams Powell**
  **11 Staple Inn**
  **London WC1V 7QH (GB)**

(54) **DELIVERY SYSTEM FOR A PRELOADED FENESTRATED DEVICE HAVING A THUMBWHEEL SHEATH DEPLOYMENT**

(57)   A preloaded stent graft delivery device (100) includes a guide wire catheter (172) having a proximal end, a distal end, and a guide wire lumen (90) therethrough; a nose cone dilator (110) at the proximal end of the guide wire catheter; a handle assembly (123) at the distal end of the guidewire catheter; a thumbwheel (133) rotatably mounted to the handle assembly, the thumbwheel having a ratchet assembly (150) disposed therein; a sheath mount (135) positioned within an interior surface of the handle assembly and coupled to the thumbwheel; a pusher catheter extending from the handle assembly towards the nose cone dilator, the pusher catheter comprising at least one lumen therethrough where the guidewire catheter extends through the at least one lumen within the pusher catheter; a sheath (106) disposed coaxially over the pusher catheter, the sheath at least partially coupled to the sheath mount within the interior surface of the handle assembly.

Figure 1

EP 3 369 402 A1

## Description

## BACKGROUND

## 1. Technical Field

This invention relates to a medical device and more particularly to a device for introduction or delivery of a stent graft or other medical device or prosthesis into the vasculature of a patient.

## 2. Background Information

It is known to introduce endovascular stent grafts into the vasculature of a patient to bridge an aneurism or damaged portion of the wall of the vasculature. In the deployment of a graft or stent graft into the human or animal body via intraluminal techniques a deployment device is used to introduce the graft into a lumen of the body and, after the graft has been deployed and expanded within the lumen, the introducer needs to be retracted.

Today, many endoluminal prostheses are radially self-expanding. Radially self-expanding prostheses are advantageous because they do not require complicated and bulky balloon catheter systems for deployment. Such prostheses present a challenge, however, in that once a prosthesis end is released and anchored into the body lumen, subsequent positioning can be difficult. This is particularly the case if the ends of the prosthesis include anchoring mechanisms to secure the prosthesis to the body lumen. As a consequence, many deployment devices have been proposed that allow the self-expanding prosthesis to be partially expanded while providing a mechanism for retaining the prosthesis ends until the prosthesis has been properly positioned.

Problems can occur, however, where the damage to the vasculature includes or is adjacent to a branch vessel from a main artery because the branch vessel may be occluded by the stent graft and cause permanent damage to the patient. Examples of such branch vessels are the renal and the mesenteric arteries extending from the aorta.

Fenestrations in a stent graft have been proposed to allow access to a branch vessel from a main stent graft but it is often necessary to provide a side branch graft to maintain access into the branch vessel. Catheterisation of such a branch vessel from a delivery device through the fenestration enables deployment of a covered stent or uncovered stent into the branch vessel. This invention provides an improved apparatus for catheterisation and deployment of side branch grafts.

## BRIEF SUMMARY

The present invention seeks to provide an improved introducer assembly, in the preferred embodiments an improved stent graft delivery device.

In one aspect of the invention, there is provided a preloaded stent graft delivery device including a guidewire catheter having a proximal end, a distal end, and a guide wire lumen therethrough; a nose cone dilator at the proximal end of the guide wire catheter; a handle assembly at the distal end of the guidewire catheter; a thumbwheel rotatably mounted to the handle assembly, the thumbwheel having a ratchet assembly disposed therein; a sheath mount positioned within an interior surface of the handle assembly and coupled to the thumbwheel; a pusher catheter extending from the handle assembly towards the nose cone dilator, the pusher catheter comprising at least one lumen therethrough where the guidewire catheter extends through the at least one lumen within the pusher catheter; a sheath disposed coaxially over the pusher catheter, the sheath at least partially coupled to the sheath mount within the interior surface of the handle assembly. In some embodiments, the pusher catheter further comprises two longitudinal auxiliary lumens. In other embodiments, the pusher catheter further comprises two longitudinal auxiliary lumens.

In another aspect of the invention, there is provided a preloaded stent graft delivery device including a guidewire catheter having a proximal end, a distal end, and a guide wire lumen therethrough; a nose cone dilator at the proximal end of the guidewire catheter; a handle assembly at the distal end of the guidewire catheter; a thumbwheel rotatably mounted to the handle assembly, the thumbwheel having a ratchet assembly disposed therein; a sheath mount positioned within an interior surface of the handle assembly and coupled to the thumbwheel; and a sheath coupled to the handle assembly and at least partially coaxially disposed over the guidewire catheter, the sheath at least partially coupled to the sheath mount within the interior surface of the handle assembly. In some embodiments, the thumbwheel is rotatable about a transverse axis. In alternative embodiments, the sheath has at least two longitudinal splits formed along a portion of a length of the sheath.

In yet another aspect of the invention, there is provided a guidewire catheter having a guidewire catheter having a proximal end, a distal end, and a guide wire lumen therethrough; a nose cone dilator at the proximal end of the guidewire catheter; a handle assembly at the distal end of the guidewire catheter; a thumbwheel rotatably mounted on an external surface of the handle assembly, the thumbwheel comprising a ratchet surface engaged with a pawl; a sheath mount radially positioned within an interior surface of the handle assembly and coupled to the thumbwheel; a pusher catheter extending from the handle assembly towards the nose cone dilator, the pusher catheter comprising at least one lumen therethrough where the guidewire catheter extends through the at least one lumen within the pusher catheter; and a sheath disposed coaxially over the pusher catheter, the sheath at least partially coupled to the sheath mount within the interior surface of the handle assembly.

BRIEF DESCRIPTION OF THE DRAWINGS

Embodiments of the present invention are described below, by way of example only, with reference to the accompanying drawings, in which:

Figure 1 shows an embodiment of a preloaded stent graft delivery device;

Figure 2 shows an embodiment of a handle assembly of the embodiment of the stent graft delivery device of Figure 1;

Figure 3 shows an embodiment of a back handle of the handle assembly of the embodiment of the stent graft delivery device of Figure 1;

Figure 4 is a cross-sectional view of the back handle of the handle assembly of the embodiment of the stent graft delivery device of Figure 1;

Figures 5A and 5B show an embodiment of the thumbwheel of the back handle of the handle assembly of the embodiment of the stent graft delivery device of Figure 1;

Figures 6A and 6B show an embodiment of a ratchet assembly associated with the embodiment of the thumbwheel of the stent graft delivery device of Figure 1;

Figures 7A and 7B illustrate an embodiment of the sheath mount and thumbwheel of the back handle of the handle assembly of the embodiment of the stent graft delivery device of Figure 1;

Figure 8 shows a cross-sectional view of part of a nose cone dilator and capsule of the stent graft delivery device of Figure 1;

Figure 9 shows a schematic detailed side view of a stent graft retained on the stent graft delivery device of Figure 1;

Figure 10 shows a transverse cross-sectional view of the pusher catheter portion of the embodiment shown in Figure 1;

Figure 11 shows a partial cross-sectional view of a handle portion of the embodiment of the stent graft delivery device of Figure 1 with the auxiliary lumens extending therefrom;

Figure 12 shows a cross-sectional view of an assembly of a manifold and pusher chatter of the embodiment of the stent graft delivery device of Figure 1; and

Figure 13A-13D shows a cross-sectional view of the trigger wire assembly of the embodiment of the stent graft delivery device of Figure 1.

DESCRIPTION OF THE PREFERRED EMBODIMENTS

The term "proximal" when referring to a delivery device refers to a direction that is farthest away from the operator using a delivery device, while the term "distal" refers to a direction that is generally closest to the operator using the delivery device. The proximal and distal ends of a delivery device can also be referred to as the introduction end of the delivery device and the operator end of the delivery device. The operator end of the delivery device is that portion of the device that is intended to remain outside of a patient during a procedure. When referring to the prosthesis itself relative to the delivery device, the proximal end of the prosthesis is that part of the prosthesis nearest the delivery end of the prosthesis delivery device and the distal end of the prosthesis is that end that is closest to the operator end of the delivery device. When referring to the prosthesis relative to placement in the human body, the ends of the various devices and parts of devices may be referred to as the inflow end (that end that receives fluid first, and the outflow end (that end from which the fluid exits). When applied to other vessels similar terms such as caudal and cranial should be understood.

The term "fenestration" means an opening provided through a surface of a prosthesis from the interior of the prosthesis to the exterior of the prostheses and may have a variety of geometries, including circular, semi-circular, oval, oblong, as well as other geometries.

The term "biocompatible" refers to a material that is substantially non-toxic in the in vivo environment of its intended use, and that is not substantially rejected by the patient's physiological system (i.e., is non-antigenic). Examples of biocompatible materials from which textile graft material can be formed include, without limitation, polyesters, such as polyethylene terephthalate; fluorinated polymers, such as polytetrafluoroethylene (PTFE) and fibres of expanded PTFE, and polyurethanes. In addition, materials that are not inherently biocompatible may be subjected to surface modifications in order to render the materials biocompatible. Examples of surface modifications include graft polymerization of biocompatible polymers on the materials surface, coating of the surface with a crosslinked biocompatible polymer, chemical modification with biocompatible functional groups, and immobilization of a compatibilizing agent such as heparin or other biocompatible substances. Thus, any fibrous material having sufficient strength to survive in the in vivo environment may be used to form a textile graft, provided the final textile is biocompatible. Fibres suitable for making textile grafts include polyethylene, polypropylene, polyaramids, polyacrylonitrile, nylon, and cellulose, in addition to the polyesters, fluorinated polymers, and polyurethanes as listed above. Furthermore, bioremodelable materials may also be used singly or in combination with the aforementioned polymer materials. The textile may be made of one or more polymers that do not require treatment or modification to be biocompatible. The graft may be constructed from woven multifilament polyester, for example and without limitation, Dacron™, produced by DuPont. Dacron™ is known to be sufficiently biologically inert, non-biodegradable, and durable to permit safe insertion inside the human body.

The term "prosthesis" means any device for insertion or implantation into or replacement for a body part or function of that body part. It may also mean a device that enhances or adds functionality to a physiological system. The term prosthesis may include, for example and without limitation, a stent, stent-graft, filter, valve, balloon, embolization coil, and the like.

The term "tubular" refers to the general shape of an endoluminal device which allows the module to carry fluid along a distance or fit within a tubular structure such as an artery. Tubular prosthetic devices include single, branched, and bifurcated devices. Tubular may refer to any shape including, but not limited to, tapered, cylindrical, curvilinear, or any combination thereof. A tubular device may have a cross-sectional shape that is, circular, substantially circular or the like. However, it should be understood that the cross-sectional shape is not limited thereto, and other shapes, such as, for example, hexagonal, pentagonal, octagonal, or the like are contemplated. The term "endoluminal" refers to or describes objects that can be placed inside a lumen or a body passageway in a human or animal body. A lumen or a body passageway can be an existing lumen or a lumen created by surgical intervention. As used in this specification, the terms "lumen" or "body passageway" are intended to have a broad meaning and encompasses any duct (e.g., natural or iatrogenic) within the human body and can include a member selected from the group comprising: blood vessels, respiratory ducts, gastrointestinal ducts, and the like. "Endoluminal device" or "endoluminal prosthesis" thus describes devices that can be placed inside one of these lumens.

The term "graft" or "graft material" describes an object, device, or structure that is joined to or that is capable of being joined to or implanted in or against a body part to enhance, repair, or replace a portion or a function of that body part. A graft by itself or with the addition of other elements, such as structural components, may comprise an endoluminal prosthesis. The graft may be comprised of a single material, a blend of materials, a weave, a laminate, or a composite of two or more materials. The graft may also be constructed from a synthetic, for example and without limitation, a polymer. The graft may be formed from a single layer or multiple layers of material. In embodiments employing a plurality of layers of material, the layers may remain separate, or may be attached to each other through a secondary process such as sintering, curing, adhesives, and sutures or the like.

The term "stent" means any device or structure that adds rigidity, expansion force or support to a prosthesis. A stent is used to obtain and maintain the patency of the body passageway while maintaining the integrity of the passageway. Also, the stent may be used to form a seal. The stent may be located on the exterior of the device, the interior of the device, or both. A stent may be self-expanding, balloon-expandable or may have characteristics of both. A variety of other stent configurations are also contemplated by the use of the term "stent." The stents 16 may be comprised of a metallic material selected from stainless steel, silver, platinum, palladium, gold, titanium, tantalum, iridium, tungsten, cobalt, chromium, cobalt-chromium alloy 1058, cobalt-based 35N alloy, nickel-based alloy 625, a molybdenum alloy, a molybdenum alloy including about 0.4% to about 0.8% of lanthanum oxide (Li2O3), and a nickel-titanium alloy, such as nitinol, or other suitable materials as known in the art. The stents may be made of a wire, or may be laser or cannula cut, or manufactured by other known methods.

Throughout this discussion the term "stent graft" is intended to mean a device which has a tubular body of biocompatible graft material and at least one stent fastened to the tubular body to define a lumen through the stent graft. The stent graft may be bifurcated and have fenestrations, side arms or the like. Other arrangements of stent grafts are also within the scope of the invention.

The terms "patient," "subject," and "recipient" as used in this application refer to any animal, especially humans.

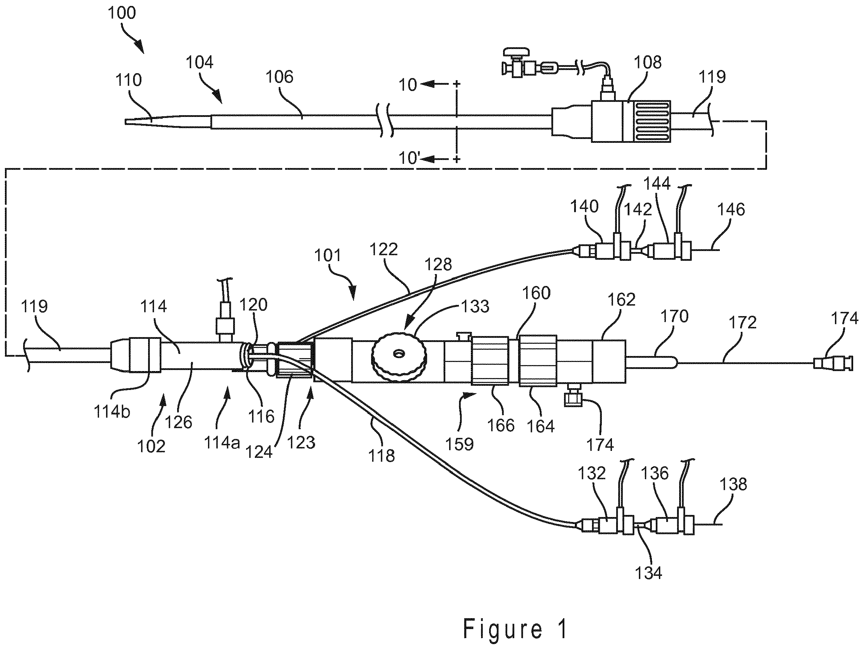

As shown in Figure 1, the delivery device 100 comprises a handle 101 and manifold assembly 102 and introduction portion 104 intended to be deployed into the patient by the known Seldinger method. More specifically the introduction portion 104 includes a sheath 106 extending from a sheath hub 108 to a nose cone dilator 110. A stent graft 131 is retained within the outer sheath 106 in the region 107 just distal of the nose cone dilator 110. A sheath hub 108 is positioned over a tri-lumen catheter 119 which extends from and is connected into a manifold 114 as is discussed in more detail below. The manifold 114 has a proximal end 114b into which is affixed the tri-lumen catheter 119 and two access ports 116, 120 at its distal end 114a.

A handle 101 comprising a handle assembly 123 for the stent graft delivery device 100 is provided. In one embodiment, a trigger wire release mechanism 159 is adjacent to the handle assembly 123. The handle assembly 123 comprises a front handle 126 and a back handle 128. The back handle 128 includes a thumbwheel 133 attached to an outer surface of the back handle 128. The manifold 114 forms part of the front handle 126. It is understood that the manifold 114 can be separate from the front handle 126 and can be disposed either proximal or distal to the front handle 126. The manifold 114 includes an access port 116 for a first access sheath 118 that extends from the manifold portion 114 of the front handle 126. Access port 120 is provided for a second access sheath 122. The access ports 116, 118 include haemostatic seals 117, 121. The first access sheath 118 extends to a haemostatic seal 132 through which extends a dilator 134. On the dilator 134 is a dilator haemostatic seal 136 through which extends an indwelling guide wire 138. Likewise, the second access sheath 122 extends to a haemostatic seal 140 through which extends a dilator 142. On the dilator 142 is a dilator haemostatic seal 144 through which extends an indwelling guide wire 146. Further, the access sheaths 118, 122 (left and right) on their respective indwelling guide wires 138, 146 will already be disposed within the lumen of stent graft 131 such that the step of advancing the access sheaths 118, 122 and guide wires 138, 146 within the lumen of the stent graft 131 after the placement of the device 100 within the patient is not required.

The trigger wire release assembly 159 includes a rotational portion 160 and a releasable portion 162. As shown, the rotational portion 160 includes a proximal thumb-handle 164 and a distal thumb-handle 166. The rotational portion is configured to be rotated about a longitudinal axis of the delivery device 10 by a user of the device 10. In this embodiment, the rotational portion may be coupled with the trigger wires associated with the stabilization retention of indwelling guide wires, a retention trigger wire for an exposed stent 137 of the stent graft 131 in the capsule 143, and the diameter reducing ties for the stent graft 131. The releasable portion of the trigger wire release assembly 159 is positioned distal to the rotational portion of the trigger wire release assembly. The releasable portion 162 of the trigger wire assembly 159 is held in place upon the trigger wire release assembly by a set screw 174. In this embodiment, the trigger wire for the distal end of the stent graft 131 is coupled to the releasable portion of the trigger wire. It is appreciated that the number of trigger wires coupled to either the rotatable portion of the trigger wire arrangement or the releasable portion of the trigger wire assembly will vary depending on the number of stents to be deployed within the vessels. A pin vice arrangement 170 is positioned at a distal end of the releasable portion of the trigger wire release assembly. The pin vice arrangement 170 locks movement of a guide wire catheter 172 with respect to the distal portion of the handle 128 and the trigger wire release assembly 159 and can be loosened to allow relative motion between these components. One of skill in the art will recognize that other embodiments may include different trigger wire arrangements.

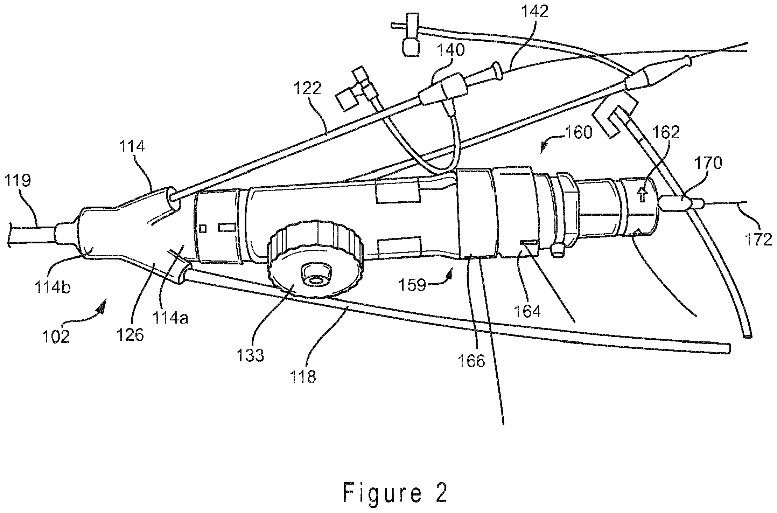

Figure 2 illustrates an embodiment of the handle assembly 123. The handle assembly 123 includes a front handle 126 and a back handle 128 disposed distal to the front handle 126. The manifold 114 forms part of the front handle 126. The manifold 114 includes an access port 116 for a first access sheath 118 that extends from the manifold portion 114 of the front handle 126. Access port 120 is provided for a second access sheath 122. The access ports 116, 118 include haemostatic seals 117, 121. The back handle 128 includes a thumbwheel 133 attached to an outer surface of the back handle 128. The thumbwheel 133 is rotatably mounted to the exterior surface of the back handle 128 and has a radially outward thumb surface. The thumbwheel 133 can rotate with respect to the front handle 126 and manifold 114. The thumbwheel 133 may be mounted to the back handle 128 via a rod 131 positioned within the interior surface of the back handle 128. A secondary sheath 178 is disposed about the sheath 106. The length of the secondary sheath 178 is approximately equal to the length of the splits 176 in the sheath 106. The function of the thin secondary sheath is to direct blood flow from the splits 176 in the sheath 106 back into the patient's body.

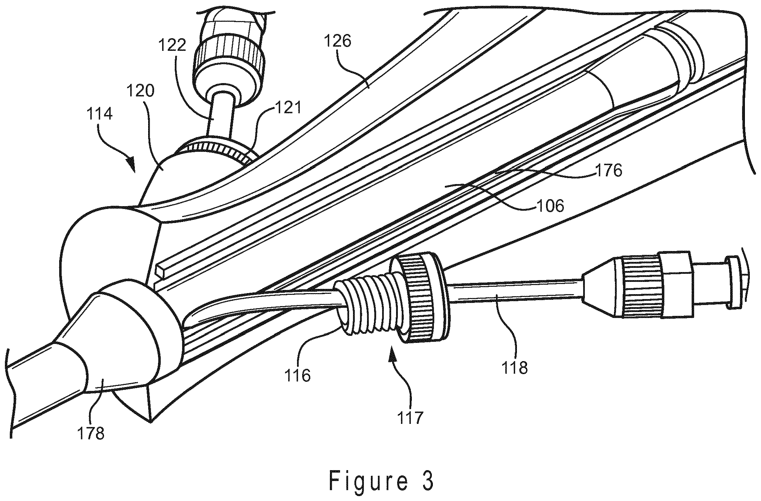

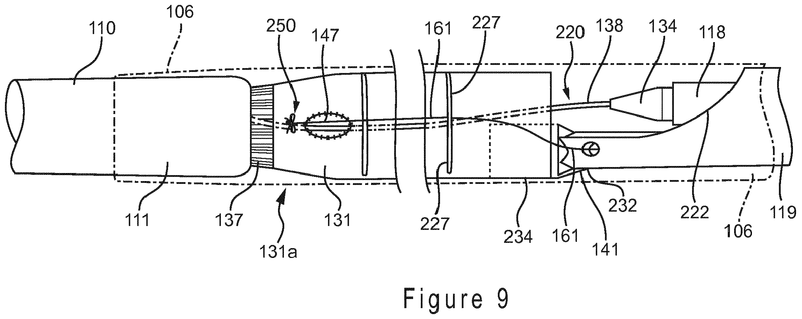

Figure 3 illustrates a transverse cross-section of the front handle. A portion of the sheath 106 includes a split 176 on either side so as to allow access into the interior of the sheath 106. The splits 176 allow for the preloading of the access sheaths 118, 122. The gap of the split 176 is sufficiently large enough to allow for the sheath 106 to be retracted while the access sheaths 118, 122 are preloaded within the manifold 114. Thus, the maximum opening of each split 176 may be equal to the outer diameter of the access sheaths 118, 122, which in one embodiment is 6 Fr. The length of each split 176 varies and can be as small as 1 mm or as long as 40 mm or greater. The length of the splits 176 may extend from a proximal end of the sheath 106 and may terminate at a location of the sheath 106 that is distal to the handle assembly 123. The splits 176 also allow the sheath 106 to be retracted, by rotation of the back handle 128, while the access sheaths 118, 122 remain in the tri lumen catheter 119. As shown in Figures 2 and 9, the splits 176 allow for continuous access to the tri-lumen catheter 119 via the apertures 210, 212 during deployment process of the main stent graft 131. This allows the main stent graft 131 to be deployed while the access sheaths 118, 122, which contain the preloaded stent grafts for the side branches, remain in the tri-lumen catheter 119. Upon deployment of the main stent graft 131, the side branch stent grafts may be inserted in the side branches in the matter described below so as to cannulated the side branches. The step of inserting the access sheaths 118, 122 into the manifold 114 after the deployment of the main stent graft 131 and the related steps are eliminated.

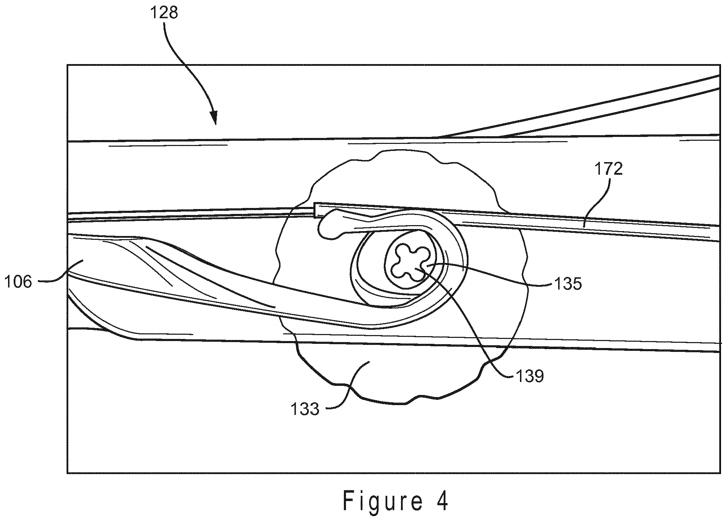

Figure 4 illustrates longitudinal cross section of the back handle 128. The back handle 128 includes a sheath mount 135 engaged with a thumbwheel 133. Disposed adjacent to the sheath mount 135 within the interior of the back handle 128 is the guide wire catheter 172 surrounded by the retrieval catheter 127. The sheath mount 135 is positioned upon a transverse axis of the back handle 128 and is disposed within the interior surface of the back handle 128. As will be discussed below, the thumbwheel 133 may include elements that are configured to mate with elements present on an end of the rod. A set screw 139 may be included to further secure the thumbwheel 133 to the sheath mount 135. A proximal end of the sheath 106 is coupled to the sheath mount 135. As shown, the sheath 106 is partially wound about the outer surface of the sheath mount 135. The sheath mount 135 serves as a spool for retracting the sheath 135 for deployment of the stent graft 131. The sheath 106 may be an elongate flexible sheath such as a Flexor® sheath commercially available from Cook Incorporated, Bloomington, Ind. In this embodiment, the proximal end of the sheath 106 may include a predetermined portion that does not include coils. The uncoiled section of the sheath 106 allows the sheath to be wrapped around the sheath mount 135. It will appreciated that the distance of the predetermined portion of the sheath 106 that does not include coils may be based on the distance the sheath 106 must be withdrawn in order to deploy the stent graft 131. In other words, the distance of the predetermined portion of the sheath may be based on the length of the stent-graft 131. The sheath 106 attached to the sheath mount 135 is in communication with the tri lumen pusher (not shown).

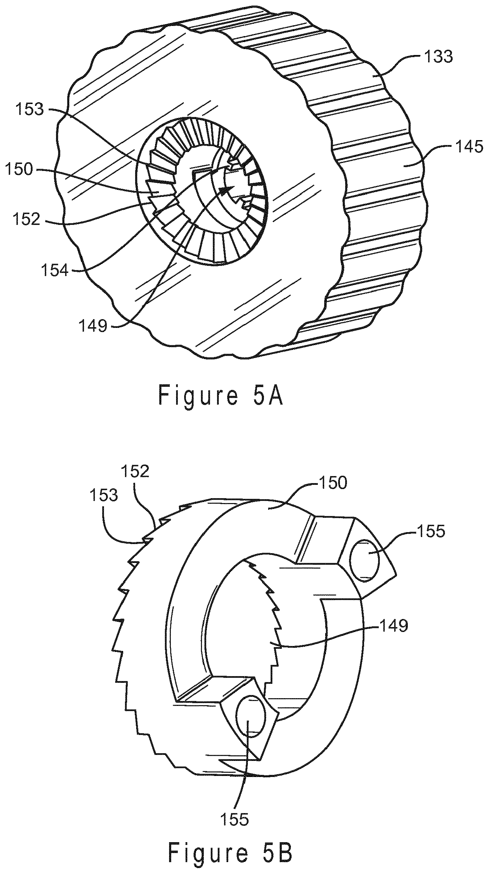

Figures 5A and 5B illustrate aspects of the thumbwheel 133. As shown in Figure 5A, the thumbwheel 133 includes an undulated, ergonomic thumb surface 145 and has a generally circular configuration. The thumbwheel 133 further includes an opening 149. Within the opening 149 of the thumbwheel, elements 154 are provided that are configured to engage corresponding elements of the sheath mount 135. Surrounding the elements 154 within the opening 149 of the thumbwheel 133 is a ratchet assembly 150 disposed within the opening 149. The ratchet assembly 150 is generally circular in configuration and includes a surface 152 comprising plurality of teeth 153 having an asymmetric configuration. Each tooth 153 has a moderate gradient on one edge and a steeper gradient on an opposite edge. The teeth 153 of the ratchet surface 152 are configured to engage with a pawl surface positioned on the back handle 128. In this embodiment, the ratchet assembly 150 is spring loaded. Referring to Figure 5B, the ratchet assembly 150 further includes a pair of openings 155 to receive the springs for the ratchet assembly 150. It will be appreciated that other suitable configurations of a ratchet assembly may be used with the thumbwheel 133.

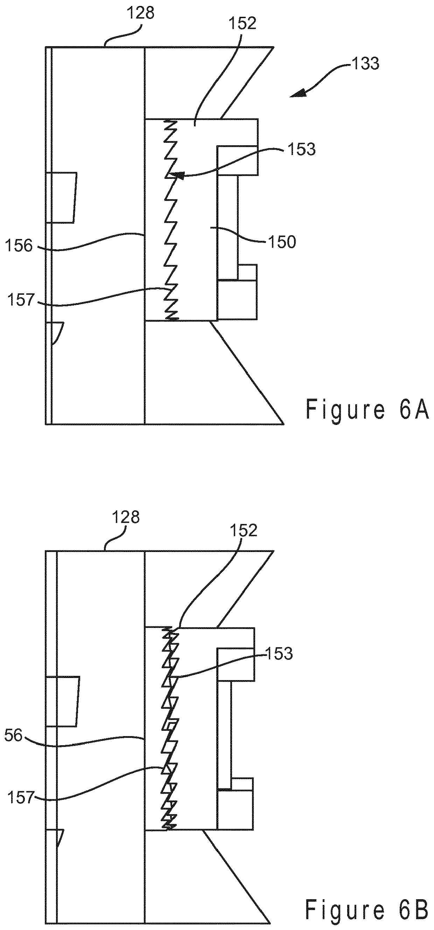

Figures 6A and 6B illustrate interaction between the ratchet assembly 150 and the pawl surface 156. As illustrated in Figure 6A, the pawl surface 156 comprising plurality of teeth 157 having an asymmetric configuration corresponding to with the teeth 153 of the ratchet surface 152 on the ratchet assembly 153. When the thumbwheel 153 is rotated in a counter-clockwise direction, the teeth 157 of the pawl 156 is configured to engage with the steep slope of the teeth 153 of the ratchet surface 152 will impede motion in that direction. Motion of the thumbwheel is also impeded by the engagement of the springs within the ratchet surface 152. Accordingly, the ratchet surface 152 of the ratchet assembly 150 impedes rotation of the thumbwheel 133 in a counter-clockwise direction. As illustrated in Figure 6B, when the thumbwheel 133 is rotated in the clockwise direction, the teeth 157 of the pawl 156 will slide within the teeth 153 of the ratchet surface 152 while simultaneously compressing the springs within the ratchet assembly 152 without restricting the natural motion of thumbwheel 133. In use, the ratchet assembly 152 prevents any movement in the proximal direction of the sheath 106 during deployment of the stent graft 131 while allowing for retraction of the sheath 106 in the distal direction.

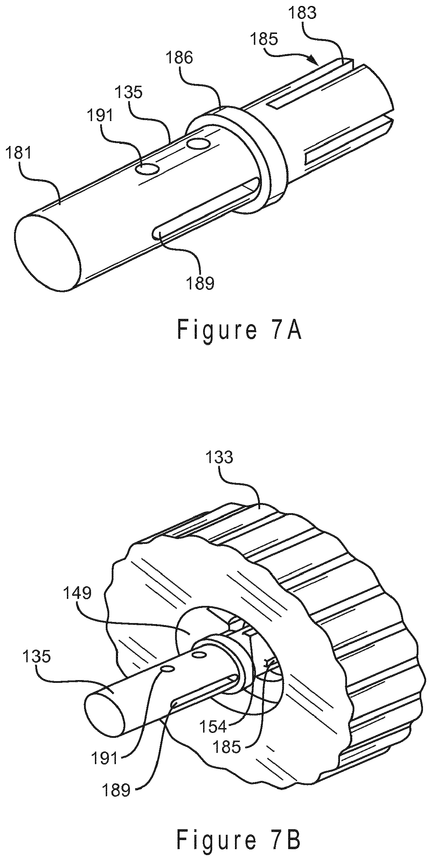

Figures 7A and 7B illustrates an embodiment of the sheath mount 135. The sheath mount 135 has a generally cylindrical shape and has a first portion 181 and a second portion 183. The first portion 181 of the sheath mount 135 includes elements 185 that mate with corresponding elements on the thumbwheel 133. A flange 186 is positioned between the first portion 181 and the second portion 183 separating the two portions of the sheath mount 135. The second section 183 includes a slot 189. The slot 189 receives a proximal portion of the sheath 106 (not shown). The rod further includes openings 191 for receiving set screws to couple the sheath 106 to the sheath mount 135. Other fastening mechanisms, such as a compression band or clip, may be used to attach these components together.

As shown in Figure 7B, the sheath mount 135 is mounted to the thumbwheel 133 through the opening 149 of the thumbwheel 133, where elements 185 mate with the corresponding elements 154 on the thumbwheel 133. When the thumbwheel 133 is rotated, the sheath 106 wraps onto the sheath mount 135 and utilize the circumference of the sheath mount 135 to achieve proper sheath travel upon deployment. Advantageously, due to the winding of the sheath 106 upon the sheath mount 135, the sheath 106 remains completely within the interior of the handle assembly 123. A mechanical advantage, or force amplification, is achieved through the use of this type of retraction mechanism for the sheath as compared to other retraction mechanisms. In particular, the mechanical advantage is based on a wheel and axle model and is calculated as a ratio of the radius of the wheel to the radius of the axle. The mechanical advantage for this aspect of the present invention is calculated as follows:

$$\frac{r_t}{r_s} = MA$$

where $r_t$ is the outer radius of the thumbwheel and $r_s$ is the outer radius of the sheath mount. This mechanical advantage reduces the amount of force required to remove the trigger wires. It will be appreciated that this ratio may be modified based on the application.

Referring back to Figure 1, trigger wire release assembly 159 includes a rotational portion 160 and a releasable portion 162. As shown, the rotational portion 160 includes a proximal thumb-handle 164 and a distal thumb-handle 166. The rotational portion is configured to be rotated about a longitudinal axis of the delivery device 10 by a user of the device 10. In this embodiment, the rotational portion may be coupled with the trigger wires associated with the stabilization retention of indwelling guide wires, a retention trigger wire for an exposed stent 137 of the stent graft 131 in the capsule 143, and the diameter reducing ties for the stent graft 131. The releasable portion of the trigger wire release assembly 159 is positioned distal to the rotational portion of the trigger wire release assembly. The releasable portion of the trigger wire assembly 159 is held in place upon the trigger wire release assembly by a set screw 174. In this embodiment, the trigger wire for the distal end of the stent graft 131 is coupled to the releasable portion of the trigger wire. It is appreciated that the number of trigger wires coupled to either the rotatable portion of the trigger wire arrangement or the releasable portion of the trigger wire assembly will vary depending on the number of stents to be deployed within the vessels. A pin vice arrangement 170 is positioned at a distal end of the releasable portion of the trigger wire release assembly. The pin vice arrangement 170 locks movement of a guide wire catheter 172 with respect to the distal portion of the handle 128 and the trigger wire release assembly 159 and can be loosened to allow relative motion between these components.

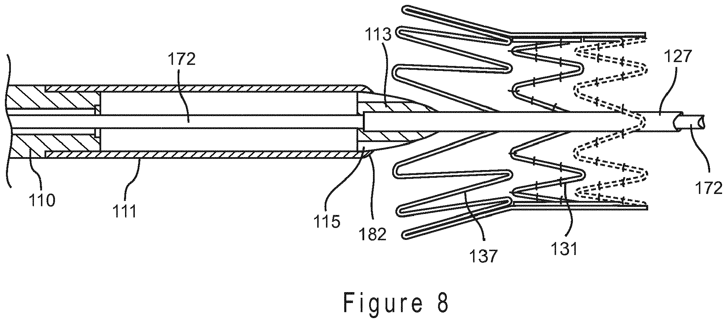

As shown Figures 1 and 8, the introduction portion 104 of the stent graft delivery device 100 includes the nose cone dilator 110 and at the distal end of the nose cone dilator 110 is a distally opening capsule 111 for the receipt of an exposed stent 137 of a stent graft 131. The capsule 111 has a slightly in-turned distal end 182. This has two purposes, a first is to assist with engagement of the sheath 106 of the delivery device when the nose cone dilator 110 is retracted into the sheath 106 and a second is to prevent complete withdrawal of a distal retrieval taper device 113 from the capsule as will be discussed below. The guide wire catheter 172 passes through and is fastened to the nose cone dilator 110 at its proximal end and passes through the handle assembly 123 of the delivery device 100.

The stent graft 131 shown in Figure 7 comprises a tubular body of a biocompatible graft material. The stent graft is supported by self-expanding stents (not shown for clarity). The proximally extending exposed stent 137 assists with providing infra-renal fixation of the deployed stent graft. The stent graft 137 is retained on the delivery device 100 by proximal retention of the exposed stent 137 into the capsule 111 of the delivery device and distally by a trigger wire release mechanism 159. Diameter reducing ties can be used to hold the stent graft in a diameter reduced condition during the initial catheterisation of a side branch because it may still be necessary to move the stent graft proximally or distally or rotate it. In the diameter reduced condition this is still possible whereas when released to full diameter this may not be possible.

United States Patent Application Ser. No. 11/507,115, filed Aug. 18, 2006 entitled "Assembly of Stent Grafts" teaches the use of diameter reducing ties for stent grafts and the teachings therein are incorporated herein in their entirety. United States Patent No. 7,435,253 entitled "Prosthesis and a Method of Deploying a Prosthesis" teaches arrangements for retaining a stent graft or prosthesis on a delivery or deployment device and allowing for independent rotational and translational movement of each end of the stent graft and the teachings therein are incorporated herein in their entirety.

As can be seen in Figure 8, the distal retrieval taper device 113 fits coaxially around the guide wire catheter 172 and can move longitudinally along the guide wire catheter 172. A retrieval catheter 127 is disposed coaxially around the guide wire catheter 172 and can move longitudinally along the guide wire catheter 172. At its proximal end, the retrieval catheter 127 may joined to the distal retrieval taper device 113 and at its distal end, the retrieval catheter 127 is joined to the front handle 126 by a suitable adhesive. The distal retrieval taper device 113 used with the embodiments of the present invention may include the retrieval taper device disclosed in United States Patent. No. 8,876,879, filed June 4, 2009 and entitled "Introducer" teaches distal retrieval taper devices (referred to therein as tapered plugs) and the teaching therein is incorporated herein in its entirety.

The distal retrieval taper device 113 has an enlarged shoulder 115 at its proximal end. The shoulder is sized so that it is of greater diameter than the smallest part of the in-turned distal end 182 of the capsule 111. By this arrangement the distal retrieval taper device 113 can move through the capsule but cannot be fully removed from the capsule. The retrieval catheter 127 is coaxial with the guide wire catheter 172. At its proximal end, the retrieval catheter 127 is affixed to the distal retrieval taper device 113 and at its distal end the retrieval catheter 127 is affixed to the front handle portion 126. This means that movement of the guide wire catheter 172 proximally with respect to the distal handle portion 128 after the release of the pin vice 170 will move the nose cone dilator 110 and capsule 111 with respect to the distal retrieval taper device 113 with the effect that the distal retrieval taper device 113 extends from the capsule thereby providing a smooth tapered surface for retrieval of the nose cone dilator through the stent graft. Locking of the pin vice after the distal retrieval taper 113 has been moved to the distal end of the capsule 111 ensures that all of the distal retrieval taper device 113, the capsule 111, the nose cone dilator 110, and the distal handle portion 129 all move together.

By this arrangement, the nose cone dilator 110 can be moved to a distal position with respect to fenestrations 147 in the stent graft 131 so that the nose cone dilator 110 and distally opening capsule 111 neither interferes with the deployment of side branch covered or uncovered stent grafts through such fenestrations 147 nor does any subsequent retraction of the nose cone dilator 110 interfere with the deployed of side branch side branch covered or uncovered stent grafts. United States Patent No. 8,118,854, filed Sep. 28, 2007 entitled "Endovascular Delivery Device" teaches apparatus and methods of deployment of stent grafts and side branch stent graft into fenestration of such stent grafts and the teaching therein is incorporated herein in its entirety. The use of the stabilization retention of the indwelling guide wire is particularly discussed therein.

Figure 9 shows detail of the stent graft 131 and its retention system in the region 107 as shown in FIG. 1. In particular there is detail shown of the distal attachment, the diameter reducing ties and the proximal retention. The stent graft 131 is retained within the sheath 106 and concentrically around the guide wire catheter 172 and retrieval catheter 127. The stent graft 131 has a fenestration 147 towards its proximal end. In use, the stent graft 131 is deployed so that the fenestration 147 is substantially aligned with a renal artery and it is intended to catheterize the renal artery through the fenestration to deploy the secondary stent graft. The secondary stent graft can be covered or uncovered side branch stent or stent graft for cannulation of the renal artery.

The stent graft 131 has a proximally extending exposed stent 137 at is proximal end 131a. In its ready to deploy condition, the proximally extending exposed stent 137 is received into the capsule 111 at the distal end of the nose cone dilator 110. At its distal end 131b the stent graft is retained to the attachment boss 220 at the proximal end of the tri-lumen catheter 119. Trigger wire 141 engages the distal end of the stent graft. Trigger wire 141 extends out of aperture 232 in the attachment boss 220 and engages into the stent graft 131 before re-entering the attachment boss through aperture 234 into the guide wire lumen 90 and exiting the guide wire lumen 90 at the proximal end of the tri-lumen catheter 119. At its distal end the trigger wire 141 is attached to the releasable handle 162.

The stent graft 131 has diameter reducing tie arrangements to retain it in a partially diameter reduced condition even after the sheath 106 has been retracted during deployment. The diameter reducing tie arrangement are on each side of the stent graft and comprise a trigger wire 161 stitched along the graft material on either side of the stent graft and loops of filament such as suture thread 227 engaged around the trigger wire and a portion of the graft material part way around the stent graft and then drawn tight. It can be appreciated that the secondary stent grafts may also be retained within the tri lumen catheter 119 in the same manner described above with respect to the stent grant 131. United States Patent Application Ser. No. 11/507,115, filed Aug. 18, 2006 entitled "Assembly of Stent Grafts" teaches apparatus and methods of diameter reduction of stent grafts and the teaching therein is incorporated herein in its entirety.

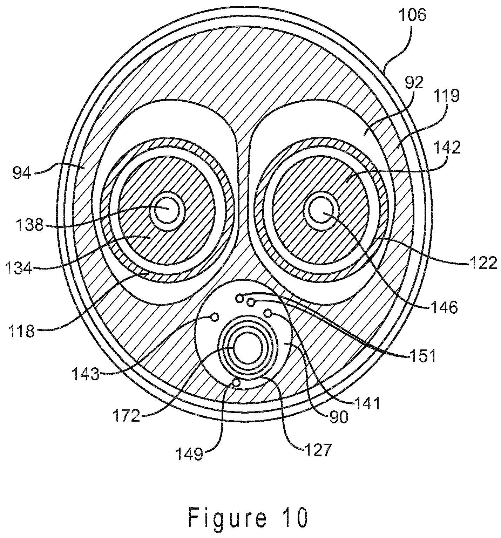

As can be seen particularly in FIG. 10, which is a transverse cross section along the line 10-10' as shown in FIG. 1, the tri-lumen catheter 119 is surrounded by the sheath 106. In this embodiment, the tri-lumen catheter 119 has three longitudinally extending lumens. A first lumen is a guide wire lumen 90 and this lumen is off-set from the centre of the tri-lumen catheter 119 to allow for two auxiliary lumens 92 and 94. The guide wire lumen 90 has passing through it the guide wire catheter 172 and coaxially around that the retrieval catheter 127.

Also in the guide wire lumen 90 are the trigger wires for the diameter reducing ties 149, the top capsule 143, the distal retention 141 and the auxiliary guide wire stabilization 151. The auxiliary lumen 94 has the first access sheath 118 extending through it and the dilator 134 and guide wire 138 extend through the first access sheath 118. The auxiliary lumen 92 has the second access sheath 122 extending through it and the dilator 142 and guide wire 146 extend through the second access sheath 122.

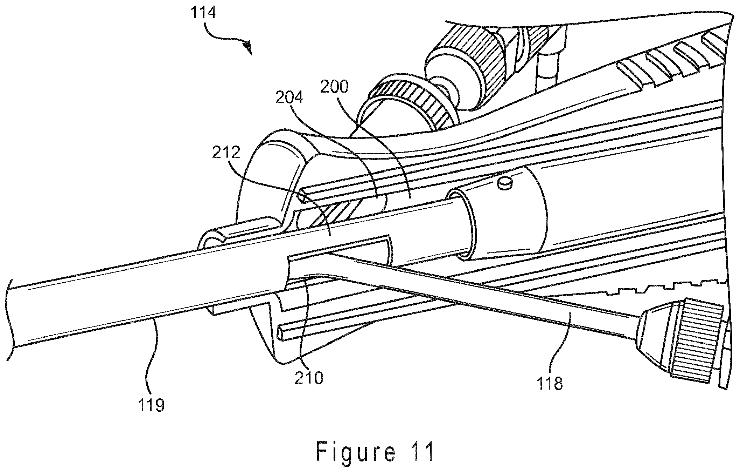

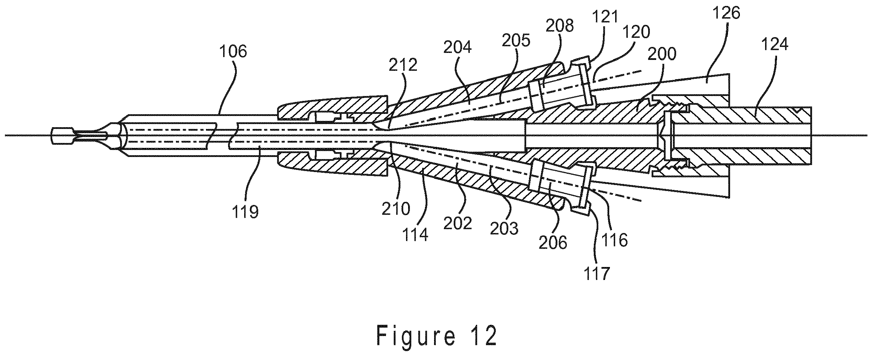

The relationship between the manifold 114, the front handle 126, and the sheath 106 is shown in more detail in Figures 11 and 12. As shown in Figures 11 and 12, the manifold 114 forms part of the front handle 126 in this embodiment and includes a through bore 200 and angled side ports 202 and 204. As can be seen in Figures 11 and 12 and 13A to 13D, the tri-lumen catheter 119 has two side apertures or windows 210 and 212 which open from the side of the tri-lumen catheter 119 into the respective lumens 92 and 94. These side apertures are elongate and tapered towards the distal end. When the tri-lumen catheter 119 is pushed into the through bore 200 of the manifold 114, the side apertures in the tri-lumen catheter 119 align with the respective angled side ports 202 and 204 of the manifold 114 thereby providing an uninterrupted lumen from the access port 116 for the first access sheath 118 into the pusher lumen 94 along the dotted line 203 and from access port 120 for a second access sheath 122 into the pusher lumen 92 along the dotted line 205 as shown in Figure 10.

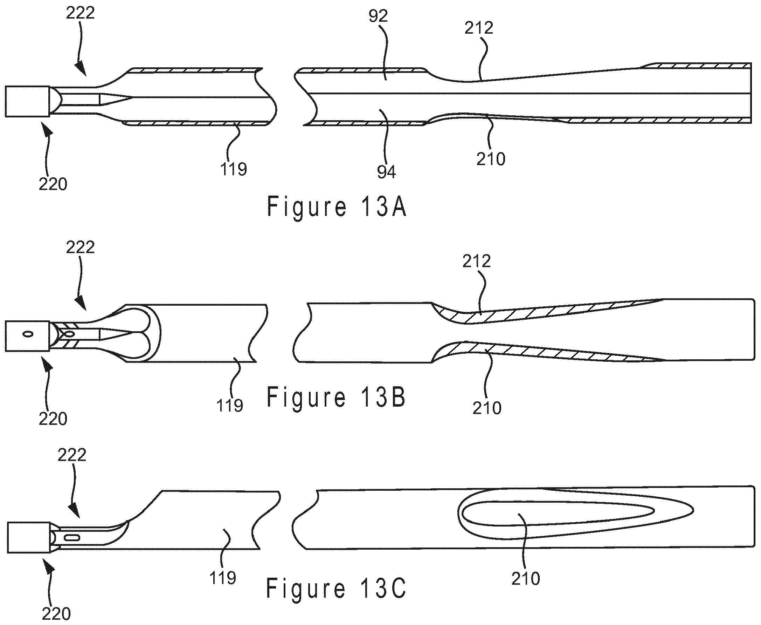

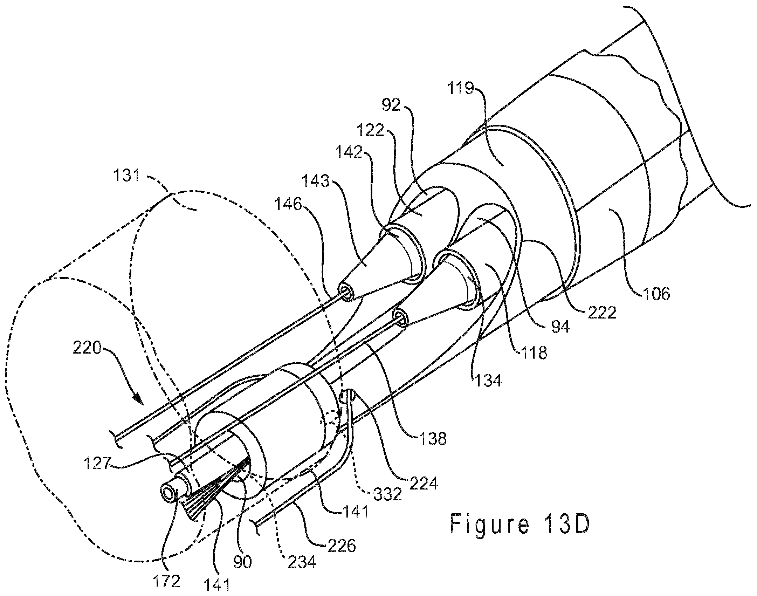

As can be best seen in FIG. 13A to 13D, at the proximal end of the tri-lumen catheter 119 is an attachment boss 220 and a scalloped end 222 to provide exit ports for the two auxiliary lumens 92 and 94. The guide wire lumen 90 opens out at the proximal end of the attachment boss 220 and to each side of the attachment boss there are apertures for trigger wires. As shown in Figure 13D, Aperture 224 is for trigger wire 226 which is used for the diameter reducing ties on one side of the stent graft 131. A corresponding aperture 228 and the other side of the attachment boss 220 is for the trigger wire 230 for the other side of the stent graft 131.

Trigger wire 141 extends out of aperture 232 in the attachment boss 220 and engages into the stent graft 131 before re-entering the attachment boss at aperture 234 and exiting the guide wire lumen 90 at the proximal end of the tri-lumen catheter 119.

Referring to Figure 11, the access sheaths (or access sheaths) 118, 122 terminate out of the renal fenestrations on top of the graft material. The access sheaths 118,122 are preloaded through the graft fenestrations and are disposed within the manifold 114, which forms part of the front handle 126. Stent grafts for stenting side branches are preloaded in the access sheaths 118, 122 so as to allow for the cannulation of side branches after the main stent graft has been deployed at the desired location. The sheath 106 is disposed between the tri-lumen catheter 119 and the bore 200. The splits 176 are formed at least partially along the length of the sheath 106 so as to allow access sheaths 118, 122 to be disposed through the splits 176 and into the tri apertures 210, 212 of the tri-lumen catheter 119. The splits 176 allow for the preloading of the access sheaths 118, 122. The gap of the split 176 is sufficiently large enough to allow for the sheath 106 to be retracted while the access sheaths 118, 122 are preloaded within the manifold 114. Thus, the maximum opening of each split 176 may be equal to the outer diameter of the access sheaths 118, 122, which in one embodiment is 6 Fr. The length of each split 176 varies and can be as small as 1 mm or as long as 40 mm or greater. The length of the splits 176 may extend from a proximal end of the sheath 106 and may terminate at a location of the sheath 106 that is distal to the handle assembly 123.

The splits 176 allow the sheath 106 to be retracted, by rotation of the back handle 128, while the access sheaths 118, 122 remain in the tri lumen catheter 119. The splits 176 allow for continuous access to the tri-lumen catheter 119 via the apertures 210, 212 during deployment process of the main stent graft 131. This allows the main stent graft 131 to be deployed while the access sheaths 118, 122, which contain the preloaded stent grafts for the side branches, remain in the tri-lumen catheter 119. Upon deployment of the main stent graft 131, the side branch stent grafts may be inserted in the side branches in the matter described below so as to cannulated the side branches. The step of inserting the access sheaths 118, 122 into the manifold 114 after the deployment of the main stent graft 131 and the related steps are eliminated.

Further, the access sheaths 118, 122 (left and right) on their respective indwelling guide wires 138 146 will already be disposed within the lumen of stent graft 131 such that the step of advancing the access sheaths 118, 122 and guide wires 138, 146 within the lumen of the stent graft 131 after the placement of the device 100 within the patient is not required.

A secondary sheath 178, shown in Figures 2 and 3, is disposed about the sheath 106. The length of the secondary sheath 178 is approximately equal to the length of the splits 176 in the sheath 106. The function of the thin secondary sheath is to direct blood flow from the splits 176 in the sheath 106 back into the patient's body

Extending out of the two auxiliary lumens 92 and 94 are the auxiliary catheters 122 and 118 respectively. From the proximal ends of the respective auxiliary catheters 118 and 122 extend dilators 134 and 142. The auxiliary guide wires 138 and 146 extend through the dilators and the secondary stents are also disposed therein

The stent graft delivery system 10 may introduced into the patient using the following steps:

1. Position the introduction part 104 of the delivery device 100 into the aorta correctly taking into account N-S position as well as rotational position with respect to target vessels and fenestrations 147 on the stent graft 131 using markers on stent graft body. At this stage the delivery device is as shown in FIGS. 1 and 2.

2. Withdraw the outer sheath 106 of the delivery device by rotating the thumbwheel 133 in the counter-clockwise direction while continuing to check position until the distal end of the stent graft 131 opens. The splits 176 of the sheath 106 widen so as to allow the sheath 106 to be retracted relative to the first and second access sheaths 118, 122 without disturbing the location of the first and second access sheaths 118, 122. At this stage the distal end of the stent graft 131 is still retained by distal fixation, the proximal end is retained by the exposed stent retained in top capsule 111 of the delivery device 100 and the expansion of the stent graft 131 is restricted by the diameter reducing ties 227.

3. Position the access sheaths 118, 122 (left and right) on their respective indwelling guide wires 138,146 at the desired location within the lumen of stent graft 131 to or through the fenestration 147 (at this stage the top capsule 111 still retains the exposed stent 137 and the indwelling guide wires).

4. Position the first access sheath 118 at the opening of the fenestration 147.

5. Remove the dilator 134 of the first access sheath 118.

6. Advance buddy wire guide (4-5 Fr) disposed in the first access sheath into the target vessel (e.g. renal artery). The additional catheter may have a crooked, curled, hockey stick tip to facilitate access.

7. Release the stabilization retention system 250 of indwelling guide wires 138 via the proximal thumb-handle 164.

8. Remove the additional catheter and replace the access sheath dilator 134 and dilator catheter over the stiffer wire in the target vessel and advance the access sheath 118, 122 over the stiffer wire into the target vessel. Withdraw the access sheath dilator.

9. Repeat steps 4 to 9 for the other of the target vessels.

10. Release the top capsule 111 by removing the locking trigger wire 143 via the distal thumbwheel 166 of the rotational portion 162 of the trigger wire release assembly 159, releasing the pin vice 170 and advancing the top capsule 111 on the guide wire catheter 100 and release the top exposed stent 137. At the same time, the distally facing capsule moves proximally over the distal retrieval taper device 113 to allow the distal retrieval taper device 113 to extend from the distal end of the capsule 111. The ratchet assembly 190 prevents rotation of the distal thumbwheel in the clockwise direction, which further prevents and proximal motion of the locking trigger wire 143. The locking trigger wire 143 is wound about the spool 182 of the distal thumbwheel 166.

11. Tighten the pin vice 170.

12. Retract the nose cone dilator 110, top capsule 111 and distal retrieval taper 113 past the fenestration 147 by removing the set screw 174 to release the releasable portion 162 of the trigger wire release assembly 159 and moving the releasable portion 162 in a distal direction. This also releases the distal attachment of the stent via trigger wire 141 connected to releasable portion 162. Retract the sheath 106 by rotating the thumbwheel 133 back handle 128.

13. One at a time, withdraw the access sheaths 118, 122 from the target vessels and deploy covered stents between the fenestrations 147 and target vessels and balloon expand if necessary including flaring within the main stent graft 131.

14. Remove both access sheaths 118, 122 and also the guide wires from the target vessels and withdraw them from the system 100.

15. Retract the nose cone dilator 110, top capsule 111 and distal retrieval taper 113 to the sheath 106.

16. Withdraw the entire assembly 100. Further deployment may include a bifurcated distal component.

The introduction steps are also disclosed in U.S. Patent No. 8,709,061, filed June 6, 2011, entitled "Preloaded Multiport Delivery Device," the entirety of which is incorporated herein by reference in its entirety.

While various embodiments of the invention have been described, the invention is not to be restricted except in light of the attached claims and their equivalents. Moreover, the advantages described herein are not necessarily the only advantages of the invention and it is not necessarily expected that every embodiment of the invention will achieve all of the advantages described.

The disclosures in US Patent Application Serial Number 62/464,476, from which this application claims priority, and in the abstract accompanying this application are incorporated herein by reference in their entirety.

**Claims**

1. A preloaded stent graft delivery device, comprising:

   a guidewire catheter having a proximal end, a distal end, and a guide wire lumen therethrough;
   a nose cone dilator at the proximal end of the guidewire catheter;
   a handle assembly at the distal end of the guidewire catheter;
   a thumbwheel rotatably mounted to the handle assembly, the thumbwheel having a ratchet assembly disposed therein;
   a sheath mount positioned within an interior surface of the handle assembly and coupled to the thumbwheel;
   a pusher catheter extending from the handle assembly towards the nose cone dilator, the pusher catheter comprising at least one lumen therethrough where the guidewire catheter extends through the at least one lumen within the pusher catheter; and,
   a sheath disposed coaxially over the pusher catheter, the sheath at least partially coupled to the sheath mount within the interior surface of the handle assembly.

2. The preloaded stent graft delivery device of claim 1, wherein the pusher catheter further comprises two longitudinal auxiliary lumens.

3. The preloaded stent graft delivery device of claim 2, wherein the pusher catheter further comprises a proximal end spaced distally from the nose cone dilator and thereby defining a stent graft retention region between the proximal end of the pusher catheter and the nose cone dilator.

4. The preloaded stent graft delivery device of claim 3, further comprising a stent being releasably retained on the stent graft retention region.

5. The preloaded stent graft delivery device of any preceding claim, wherein the ratchet assembly is disposed in an opening of the thumbwheel.

6. The preloaded stent graft delivery device of claim 5, wherein the handle assembly further includes a manifold.

7. A preloaded stent graft delivery device, comprising:

   a guidewire catheter having a proximal end, a distal end, and a guide wire lumen therethrough;
   a nose cone dilator at the proximal end of the guidewire catheter;
   a handle assembly at the distal end of the guidewire catheter;
   a thumbwheel rotatably mounted to the handle assembly, the thumbwheel having a ratchet assembly disposed therein;
   a sheath mount positioned within an interior surface of the handle assembly and coupled to the thumbwheel; and
   a sheath coupled to the handle assembly and at least partially coaxially disposed over the guidewire catheter, the sheath at least partially coupled to the sheath mount within the interior surface of the handle assembly.

8. The preloaded stent graft delivery device of any preceding claim, wherein the ratchet assembly comprises a ratchet surface and a pawl engaged with the ratchet surface.

9. The preloaded stent graft delivery device of any preceding claim, wherein the thumbwheel has an outer diameter and the sheath mount has an outer diameter lesser than the outer diameter of the thumbwheel.

10. A preloaded stent graft delivery device, comprising:

a guidewire catheter having a proximal end, a distal end, and a guide wire lumen therethrough;

a nose cone dilator at the proximal end of the guidewire catheter;

a handle assembly at the distal end of the guidewire catheter;

a thumbwheel rotatably mounted on an external surface of the handle assembly, the thumbwheel comprising a ratchet surface engaged with a pawl;

a sheath mount radially positioned within an interior surface of the handle assembly and coupled to the thumbwheel;

a pusher catheter extending from the handle assembly towards the nose cone dilator, the pusher catheter comprising at least one lumen therethrough where the guidewire catheter extends through the at least one lumen within the pusher catheter; and,

a sheath disposed coaxially over the pusher catheter, the sheath at least partially coupled to the sheath mount within the interior surface of the handle assembly.

11. The preloaded stent graft delivery device of any preceding claim, wherein the thumbwheel is rotatable about a transverse axis.

12. The preloaded stent graft delivery device of any preceding claim, wherein the sheath has at least two longitudinal splits formed along a portion of a length of the sheath.

13. The preloaded stent graft delivery device of any preceding claim, wherein the sheath mount includes an opening for receiving a distal end of the sheath.

Figure 1

EP 3 369 402 A1

Figure 2

EP 3 369 402 A1

Figure 3

Figure 4

Figure 5A

Figure 5B

Figure 6A

Figure 6B

Figure 7A

Figure 7B

Figure 8

Figure 9

EP 3 369 402 A1

Figure 10

Figure 11

Figure 12

Figure 13A

Figure 13B

Figure 13C

Figure 13D

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

Application Number

EP 18 27 5026

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/297378 A1 (SENNESS CHARLES [US] ET AL) 22 October 2015 (2015-10-22) * paragraphs [0041] - [0047]; figures 1-17 * ----- | 7-9 | INV. A61F2/966 A61F2/95 |
| X | WO 2016/063593 A1 (KANEKA CORP [JP]) 28 April 2016 (2016-04-28) * figures 1-4 * ----- | 7-9 | |

**TECHNICAL FIELDS SEARCHED (IPC)**

A61F

## INCOMPLETE SEARCH

The Search Division considers that the present application, or one or more of its claims, does/do
not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| The Hague | 30 July 2018 | Geuer, Melanie |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

                                                    
& : member of the same patent family, corresponding
document

EPO FORM 1503 03.82 (P04E07)

Europäisches
Patentamt
European
Patent Office
Office européen
des brevets

**INCOMPLETE SEARCH
SHEET C**

Application Number

EP 18 27 5026

Claim(s) completely searchable:
       7-9

Claim(s) not searched:
       1-6, 10-13

Reason for the limitation of the search:

The search has been restricted to the subject-matter indicated by the applicant in his letter of 19.07.218 filed in reply to the invitation pursuant to Rule 62a(1) EPC, that means independent claim 7 and its dependent claims 8 and 9

## ANNEX TO THE EUROPEAN SEARCH REPORT
## ON EUROPEAN PATENT APPLICATION NO.

EP 18 27 5026

This annex lists the patent family members relating to the patent documents cited in the above-mentioned European search report.
The members are as contained in the European Patent Office EDP file on
The European Patent Office is in no way liable for these particulars which are merely given for the purpose of information.

30-07-2018

| Patent document cited in search report | | Publication date | Patent family member(s) | | Publication date |
|---|---|---|---|---|---|
| US 2015297378 | A1 | 22-10-2015 | CN | 107072801 A | 18-08-2017 |
| | | | EP | 3131507 A2 | 22-02-2017 |
| | | | JP | 2017511201 A | 20-04-2017 |
| | | | US | 2015297378 A1 | 22-10-2015 |
| | | | WO | 2015160873 A2 | 22-10-2015 |
| WO 2016063593 | A1 | 28-04-2016 | JP | WO2016063593 A1 | 14-09-2017 |
| | | | US | 2017216063 A1 | 03-08-2017 |
| | | | WO | 2016063593 A1 | 28-04-2016 |

EPO FORM P0459

For more details about this annex : see Official Journal of the European Patent Office, No. 12/82

**EP 3 369 402 A1**

**Patent documents cited in the description**

- US 11507115 B
- US 7435253 B
- US 8876879 B
- US 8118854 B
- US 8709061 B
- US 62464476 A